# EUROPÄISCHE PATENTSCHRIFT

(11) **EP 2 563 899 B1**
(45) Veröffentlichungstag und Bekanntmachung des Hinweises auf die Patenterteilung: **08.03.2017**
(21) Anmeldenummer: 11716378.2
(22) Anmeldetag: 21.04.2011
(51) Int. Cl.: C12M 1/00, C12M 1/12, C12M 3/06

(54) **BIOREAKTORANORDNUNG, SCHÜTTELVORRICHTUNG UND VERFAHREN ZUM BESTRAHLEN EINES MEDIUMS IN EINEM BIOREAKTOR**
BIOREACTOR ASSEMBLY, AGITATING DEVICE AND METHOD FOR IRRADIATING A MEDIUM IN A BIOREACTOR
SYSTÈME DE BIORÉACTEUR, DISPOSITIF D'AGITATION ET PROCÉDÉ POUR L'EXPOSITION À UN RAYONNEMENT D'UN MILIEU DANS UN BIORÉACTEUR

(30) Priorität: 28.04.2010 DE 102010018678
(43) Veröffentlichungstag der Anmeldung: 06.03.2013
(73) Patentinhaber: Sartorius Stedim Biotech GmbH, 37079 Göttingen (DE)
(72) Erfinder: RÖLL, Marcel, CH-8317 Tagelswangen (CH); BÖTTCHER, Lars, 34212 Melsungen (DE)
(86) Internationale Anmeldenummer: PCT/EP2011/002044
(87) Internationale Veröffentlichungsnummer: WO 2011/134629

(56) Entgegenhaltungen:
- EP-B1- 1 173 542
- WO-A1-2010/016538
- DE-B3-102008 010 780
- DE-U1-202007 013 405

## Beschreibung

Die Erfindung betrifft eine Bioreaktoranordnung, mit einem Bioreaktor und einem Beleuchtungskörper zur Beleuchtung eines in einem Reaktorinnenraum angeordneten Mediums, wobei der Beleuchtungskörper mindestens eine Abstrahlfläche aufweist über die elektromagnetische Strahlung, wie Licht, abgestrahlt wird, die über mindestens eine Stirnfläche in den Reaktorinnenraum eingespiegelt wird.
Die Erfindung betrifft weiterhin eine Schüttelvorrichtung mit einer in Schwingungen versetzbaren Aufnahme für einen als Behälter mit einer transparenten Wandung ausgebildeten Bioreaktor.
Die Erfindung betrifft weiterhin ein Verfahren zum Bestrahlen eines Mediums in einem Bioreaktor mit transparenter Wandung mit elektromagnetischer Strahlung, wie Licht, der in einer Schüttelvorrichtung in Schüttelbewegungen versetzt wird.

Für die Kultivierung von phototrophen Zellkulturen in Bioreaktoren besitzt neben den Umgebungsbedingungen, wie z.B. Temperatur, Feuchtigkeit und CO₂-Gehalt, die Beleuchtung eine entscheidende Bedeutung.
Aus der DE 298 19 259 U1 ist eine Bioreaktoranordnung bekannt, die einen Photobioreaktor aufweist, in dessen Reaktorinnenraum ein zylindrischer Beleuchtungskörper angeordnet ist. Aus einer außerhalb des Reaktorinnenraumes angeordneten Lichtquelle wird über einen die Reaktorwandung durchdringenden Lichtleiter, der in dem Reaktorinnenraum ringförmig aufgefächert ist, Licht in die Stirnfläche des Beleuchtungskörpers eingespiegelt, das dann seitlich über die Zylinderflächen des Beleuchtungskörpers abgestrahlt wird. Zur seitlichen Abstrahlung weist der transparente Beleuchtungskörper Einschlüsse bzw. Partikel auf. Zur Unterstützung der seitlichen Abstrahlung können die Seitenflächen des Beleuchtungskörpers zudem eine angeraute Oberfläche aufweisen. Nachteilig bei der bekannten Bioreaktoranordnung ist, dass zur Einbringung des Lichtleiters die Reaktorwandung durchbrochen werden muss. Dies führt zu einer relativ aufwendigen und kostenintensiven Ausbildung des Bioreaktors. Ein derartiger Bioreaktor ist zudem nicht geeignet, um als zusammenfaltbarer, beutelförmiger Einwegbioreaktor verwendet zu werden.
Weiterhin ist aus der WO 2010/016538 A1 eine Photoreaktoranordnung bekannt, bei der in dem Reaktorinnenraum ebenfalls ein Beleuchtungskörper angeordnet ist, über dessen Stirnfläche Licht eingespiegelt und über seitliche Abstrahlflächen abgestrahlt wird. Die Lichteinspiegelung erfolgt dabei über den Stirnflächen vorgelagerte LEDs. Auch diese Bioreaktoranordnung weist die oben genannten Nachteile auf.
Aus der WO 2009/069967 A2 ist ein quader- oder zylinderförmig ausgebildeter Photobioreaktor bekannt, in dessen Reaktorinnenraum lichtabstrahlende ebene oder zylinderförmige Beleuchtungskörper angeordnet sind. In die Beleuchtungskörper wird Licht aus LEDs eingekoppelt. Hierzu ist es notwendig, die LEDs einschließlich ihrer Platinen flüssigkeitsdicht zu versiegeln. Auch diese Bioreaktoranordnung weist den Nachteil auf, dass sie relativ aufwendig und kostenintensiv ist. Auch ist diese Bioreaktoranordnung nicht für die Verwendung von flexiblen Einwegbioreaktoren geeignet.

Die DE 20 2007 013405 U1 beschreibt eine Beleuchtungseinrichtung für Laborgefäße, deren Unterseite lichtdurchlässig ist. Die lichtdurchlässige Unterseite der Laborgefäße ist jener Seite eines Leuchtdiodenträgers zugewandt, die mit mehreren Leuchtdioden versehen ist. Der Leuchtdiodenträger ist dazu in einer Laborgefäßhalterung unterhalb der Laborgefäße integriert. Der Leuchtdiodenträger kann in einer gedichteten Einhausung untergebracht sein, wozu zwischen Laborgefäßhalterung und Leuchtdiodenträger eine Scheibe aus beispielsweise Polycarbonat oder Glas angeordnet ist. Nachteilig ist, dass Maßnahmen zur Wärmeabfuhr erforderlich sind, wie Beabstandung des Leuchtdiodenträgers von den Laborgefäßböden und Ausbildung des Leuchtdiodenträgers als metallische Leiterplatte mit aktiver Kühlung des Leuchtdiodenträgers.

Die DE 10 2008 010780 B3 offenbart einen Inkubator mit einem Inkubationsraum, einer von einem Antrieb antreibbaren Schüttelvorrichtung zum Schütteln von in dem Inkubationsraum anordenbaren Gefäßen mit Zellkulturen und einem dem Inkubationsraum benachbarten Vorrichtungsraum zur Aufnahme mindestens von Teilen der in den Inkubationsraum hineinragenden Schüttelvorrichtung. Die Schüttelvorrichtung weist eine Grundplatte auf, die den Inkubationsraum gegenüber dem Vorrichtungsraum abdichtet und auf ihrer dem Inkubationsraum zugewandten Innenseite einen von einem Antriebsarm in einer horizontalen Ebene beweglichen Schütteltisch. Auf der der Innenseite abgewandten Außenseite der Grundplatte ist ein Motor angeordnet, über den eine in der Grundplatte rotierend gelagerte Antriebsachse antreibbar ist, die mit dem Antriebsarm in einer Wirkverbindung steht. Eine Bestrahlung der Zellkulturen in den Gefäßen ist nicht vorgesehen. Schließlich sind beispielsweise aus der DE 44 32 515 A1 und der DE 10 2008 28 497 A1 Beleuchtungskörper in Form von Lichtleitplatten bekannt.
Um die notwendige Begasung und den notwenigen Nährstofftransfer des im Bioreaktor befindlichen Mediums bzw. der Zellkultur sicher zu stellen, werden in den Bioreaktoren Mischer bzw. Rührer eingesetzt. Insbesondere bei Bioreaktoren, die als flexible Einwegbehälter verwendet werden, werden zum Mischen Schüttelvorrichtungen eingesetzt, die den Bioreaktor mit dem enthaltenen Medium schütteln bzw. in Vibrations- oder Schwenkbewegungen versetzen.
So ist aus der EP 1 173 542 B1 eine Schüttelvorrichtung bekannt, die eine in Schwingungen versetzbare Reaktoraufnahme für einen flexiblen Behälter aufweist. Nachteilig bei dieser Vorrichtung, die sich grundsätzlich bewährt hat, ist, dass sie nicht in Verbindung mit Pflanzenzellkulturen als Medium in Photobioreaktoren eingesetzt werden kann, die eine definierte Belichtung benötigen. Dabei soll das klimatische System im Reaktorinnenraum nicht durch einen Eintrag von Wärme, beispielsweise durch von einer Lichtquelle erzeugte Wärme, gestört werden.

Aufgabe der vorliegenden Erfindung ist es daher, eine Bioreaktoranordnung mit einem Bioreaktor anzugeben, der geeignet ist, um als Photoreaktor zur Bestrahlung eines Mediums mit elektromagnetischer Strahlung, insbesondere Licht, eingesetzt zu werden.
Weitere Aufgabe der Erfindung ist es, eine Schüttelvorrichtung für einen insbesondere als flexiblen Behälter ausgebildeten Photoreaktor anzugeben.
Weitere Aufgabe der Erfindung ist es, ein Verfahren zum Bestrahlen eines Mediums in einem Bioreaktor mit einer transparenten Wandung anzugeben, bei dem der Bioreaktor in einer Schüttelvorrichtung durchmischt werden kann.

Die Aufgabe bezüglich der Bioreaktoranordnung wird in Verbindung mit dem Oberbegriff des Anspruches 1 dadurch gelöst, dass der Bioreaktor als ein Behälter mit einer transparenten Wandung ausgebildet ist, dass der Beleuchtungskörper außerhalb des Reaktorinnenraumes benachbart zu mindestens einem Teilbereich der transparenten Wandung angeordnet ist, und dass der Beleuchtungskörper mit seiner Abstrahlfläche eine Auflage- oder Stützfläche für den Bioreaktor bildet.
Durch die Anordnung des Beleuchtungskörpers außerhalb des Reaktorinnenraums wird es ermöglicht, den Bioreaktor als einen flexiblen Behälter mit einer transparenten Wandung auszubilden. Dadurch wird es außerdem möglich, den flexiblen Behälter bzw. Bioreaktor zusammengefaltet und steril verpackt zu liefern. Durch die Ausbildung der Abstrahlfläche des Beleuchtungskörpers als Auflage- oder Stützfläche für den Bioreaktor, kann der Bioreaktor einfach auf dem Beleuchtungskörper abgelegt werden und weist mit seiner die Abstrahlfläche kontaktierenden Wandung zwangsläufig eine definierte Belichtungsposition zum Beleuchtungskörper auf. Durch die Einstrahlung bzw. Einspiegelung elektromagnetischer Strahlung der für die Reaktion erforderlichen Wellenlängen, insbesondere von Licht, in die Stirnfläche des Beleuchtungskörpers erfolgt praktisch kein Wärmeeintrag in den Reaktorinnenraum.
Der Beleuchtungskörper weist in einer bevorzugten Ausführungsform Aussparungen zur Durchführung von Sensoren, Anschlüssen oder Rührern zum Bioreaktor auf.
Gemäß einer bevorzugten Ausführungsform der Erfindung ist der Beleuchtungskörper an einer Reaktoraufnahme einer Schüttelvorrichtung angeordnet. Durch die Anordnung des Beleuchtungskörpers an der Reaktoraufnahme der Schüttelvorrichtung bildet dessen Abstrahlfläche die Auflage- oder Stützfläche der Reaktoraufnahme der Schüttelvorrichtung. Damit ist es relativ einfach möglich, eine an sich bekannte Schüttelvorrichtung nunmehr für einen als Behälter, der beispielsweise über flexible Wände verfügt, ausgebildeten Bioreaktor zu verwenden, der durch den Beleuchtungskörper zu einem Photobioreaktor wird.
Nach einer weiteren bevorzugten Ausführungsform der Erfindung ist der Bioreaktor als ein flexibler Kunststoffbeutel zum Einmalgebrauch ausgebildet. Durch die Anordnung des Beleuchtungskörpers außerhalb des Bioreaktors kann der Bioreaktor als ein kostengünstiger flexibler Kunststoffbeutel zum Einmalgebrauch ausgebildet sein bzw. verwendet werden.

Erfindungsgemäß ist der Beleuchtungskörper als eine ebene Platte ausgebildet, wobei an mindesten einer Stirnfläche zur Einspiegelung von elektromagnetischen Strahlung, insbesondere Licht, eine Lichtleiste mit einer Mehrzahl von definierte Wellenlängen abstrahlenden Leuchtkörpern, insbesondere LED-Lichtquellen angeordnet ist. Durch die Ausbildung als ebene Platte kann der Beleuchtungskörper relativ einfach und kostengünstig hergestellt werden. Grundsätzlich ist es aber auch möglich, den Beleuchtungskörper an die Form des Bioreaktors anzupassen. Auch ist es beispielsweise möglich, den Beleuchtungskörper zylindrisch oder topfförmig auszubilden, so dass die Wandung des Bioreaktors durch den Beleuchtungskörper abgestützt wird. Dies ermöglicht insbesondere den Bioreaktor statt kissenförmig zylindrisch auszubilden. Durch die Verwendung einer Mehrzahl von LED-Lichtquellen entsteht zudem außerhalb des Reaktorinnenraumes nur eine geringe, vernachlässigbare Wärmeentwicklung der Lichtquellen.
Nach einer weiteren bevorzugten Ausführungsform der Erfindung weist der Beleuchtungskörper in seinem Inneren lichtstreuende Partikel auf. Die lichtstreuenden Partikel führen dazu, dass die sonst übliche Totalreflexion in dem Beleuchtungskörper unterbrochen und über die Stirnseiten eingespiegeltes Licht seitlich aus der Abstrahlfläche austritt. Die dem Bioreaktor abgewandte Fläche kann dabei verspiegelt sein, um einen Lichtaustritt auf dieser Seite zu verhindern. Auch ist es möglich, die Abstrahlfläche mit einer lichtstreuenden Struktur zu versehen. Die lichtstreuende Struktur kann beispielsweise aus einer aufgerauten Oberfläche bestehen.

Die Aufgabe bezüglich der Schüttelvorrichtung wird in Verbindung mit dem Oberbegriff des Anspruchs 9 dadurch gelöst, dass die Reaktoraufnahme einen flächigen Beleuchtungskörper aufweist, dessen Abstrahlfläche für den aufzunehmenden Bioreaktor eine Auflage- oder Stützfläche bildet, über die elektromagnetische Strahlung, wie Licht, in den Reaktorinnenraum des Bioreaktors einspiegelbar ist.
Durch die Anordnung eines flächigen Beleuchtungskörpers in der Reaktoraufnahme für den Bioreaktor ist es auf einfache Art möglich, elektromagnetische Strahlung, insbesondere Licht, in den Reaktorinnenraum des Bioreaktors einzuspiegeln, so dass der Bioreaktor während des Schüttelvorganges als Photoreaktor genutzt werden kann. Erfindungsgemäß wird
über die Abstrahlfläche elektromagnetische Strahlung, wie Licht, in den Bioreaktor abgestrahlt, die über mindestens eine Stirnfläche einspiegelbar ist. Die Einspiegelung in den Bioreaktor erfolgt dabei praktisch wärmefrei, so dass das Wärmeklima des Reaktorinnenraumes nicht beeinflusst wird.

Durch die Einspiegelung über die Stirnfläche bzw. die Stirnflächen des Beleuchtungskörpers wird die Wärmentwicklung der Lichtquellen weitgehend an die Umgebung des Bioreaktors abgegeben.

Die Aufgabe bezüglich des Verfahrens wird in Verbindung mit dem Oberbegriff des Anspruchs 10 dadurch gelöst, dass der Bioreaktor in eine Reaktoraufnahme der Schüttelvorrichtung so eingesetzt wird, dass mindestens ein Teil seiner Wandung an einer von einer Abstrahlfläche eines flächigen Beleuchtungskörpers gebildeten Auflage- oder Stützfläche der Reaktoraufnahme anliegt, und dass in mindestens eine Stirnseite des Beleuchtungskörpers elektromagnetische Strahlen, insbesondere Licht, eingespiegelt wird, die über die Abstrahlfläche und die Wandung des Bioreaktors in das in dem Reaktorinnenraum angeordnete Medium einstrahlt.
Dadurch, dass Leuchtkörper eingesetzt werden, die elektromagnetische Strahlung definierter Wellenlängen liefern, ist eine breite Nutzung des Verfahrens beim Anwender möglich. In Abhängigkeit von der Kultivierungsaufgabe, kann die Wellenlänge und die Intensität der Strahlung geregelt werden. Dabei hat sich die Regelung der Lichtstärke über die Erfassung der Biomassebildung besonders bewährt.
Das erfindungsgemäße Verfahren ermöglicht auf einfache und kostengünstige Weise das Einstrahlen von Licht in ein Medium mit phototropen Mikroorganismen oder Algen eines Photoreaktors, wobei eine gleichzeitige Durchmischung des Mediums in einer Schüttelvorrichtung ermöglicht wird. Weitere Einzelheiten der Erfindung ergeben sich aus der nachfolgenden ausführlichen Beschreibung und den beigefügten Zeichnungen, in denen bevorzugte Ausführungsformen der Erfindung beispielhaft veranschaulicht sind.

In den Zeichnungen zeigen:
- Figur 1:: eine Seitenansicht einer Bioreaktoranordnung teilweise im Schnitt mit einer Schüttelvorrichtung,
- Figur 2:: eine Seitenansicht der Reaktoraufnahme der Schüttelvorrichtung von Figur 1 ohne Bioreaktor in vergrößerter Darstellung,
- Figur 3:: eine Seitenansicht des Beleuchtungskörper von Figur 1 im Schnitt und
- Figur 4:: eine Draufsicht auf den Beleuchtungskörper von Figur 3 aus Richtung IV.

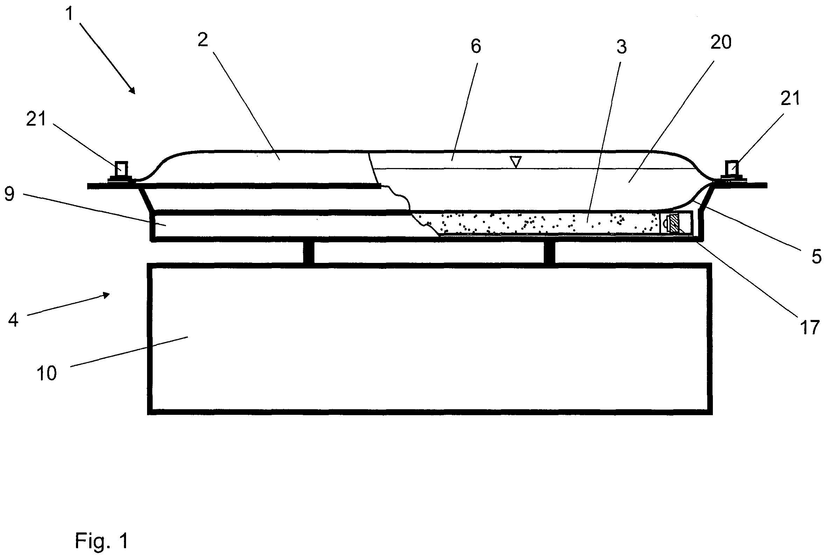

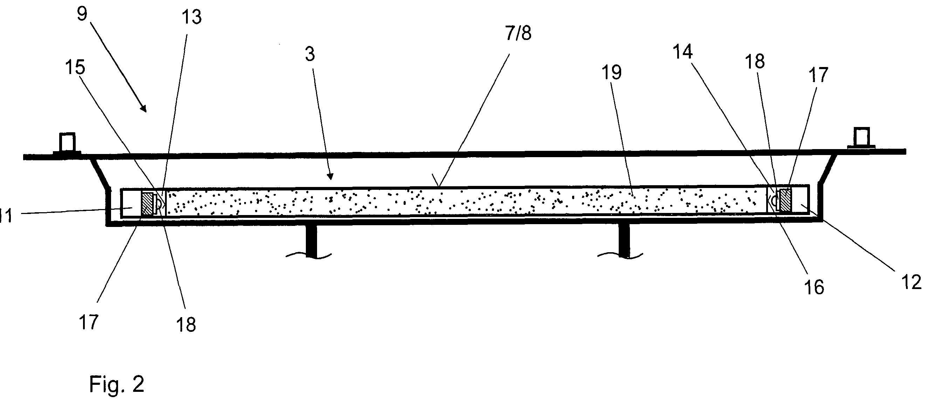

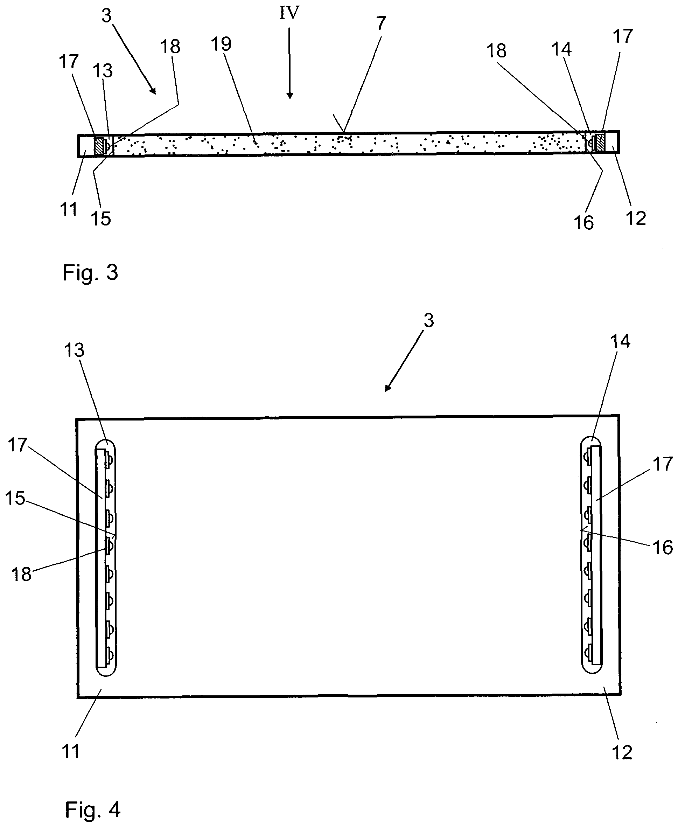

Eine Bioreaktoranordnung 1 besteht im Wesentlichen aus einem Bioreaktor 2, einem Beleuchtungskörper 3 und einer Schüttelvorrichtung 4.
Der Bioreaktor 2 ist entsprechend dem Ausführungsbeispiel von Figur 1 als ein flexibler beutelförmiger Behälter mit einer transparenten Wandung 5 ausgebildet, die einen Reaktorinnenraum 6 umschließt. Dem Fachmann bekannte Zu- und Abflüsse zu dem Reaktorinnenraum 6 wurden zur besseren Übersicht nicht dargestellt. Der Beleuchtungskörper 3 bildet mit seiner dem Bioreaktor 2 zugewandten Abstrahlfläche 7 eine Auflagefläche 8 einer Reaktoraufnahme 9 der Schüttelvorrichtung 4. Die Schüttelvorrichtung 4 weist ein Antriebs- und Steuergerät 10 auf, das die Reaktoraufnahme 9 in Schüttelbewegungen bzw. Schwingungen versetzt. Entsprechend den Figuren 1 bis 4 ist der Beleuchtungskörper 3 als eine ebene Platte ausgebildet. An seinen stirnseitigen Enden 11, 12 weist der Beleuchtungskörper 3 zwei quer verlaufende Durchbrüche 13, 14 auf, die die funktionsmäßigen Stirnflächen 15, 16 freilegen, über die Licht in den Beleuchtungskörper 3 eingespiegelt wird. In die Durchbrüche 13, 14 wird jeweils eine Lichtleiste 17 mit LED-Lichtquellen 18 eingesetzt. Das Licht der LED-Lichtquellen 18 wird über die Stirnflächen 15, 16 des Beleuchtungskörpers 3 eingespiegelt und über die dem Bioreaktor 2 zugewandte Abstrahlfläche 7 abgestrahlt. In seinem Inneren weist der Beleuchtungskörper 3 lichtstreuende Partikel 19 auf, die die seitliche Abstrahlung über die Abstrahlfläche 7 unterstützen sollen. Zusätzlich kann die Abstrahlfläche 7 eine lichtstreuende Struktur, beispielsweise in Form einer aufgerauten Oberfläche aufweisen.
Im Ausführungsbeispiel ist der Beleuchtungskörper 3 aus einem Acrylglas ausgebildet. Generell kann der Beleuchtungskörper 3 aus einem beliebigen lichtabstrahlenden Material gebildet sein, beispielsweise auch Glas.
Das über die Abstrahlfläche 7 abgestrahlte Licht wird durch die transparente Wandung 5 des Bioreaktors 2 in das im Reaktorinnenraum 6 befindliche Medium 20, das die Zellkultur enthält, eingestrahlt.
Der das Medium 20 enthaltende beutelförmige Bioreaktor 2 wird in die Reaktoraufnahme 9 der Schüttelvorrichtung 4 so eingesetzt, dass der der Abstrahlfläche 7 des Beleuchtungskörpers 3 zugewandte Teil seiner transparenten Wandung 5 an der Abstrahlfläche 7, die die Auflagefläche 8 der Reaktoraufnahme 9 bildet, anliegt. Dabei wird das in die Stirnflächen 15, 16 des Beleuchtungskörpers 3 eingespiegelte Licht der Lichtleisten 17 mit ihren LED-Lichtquellen 18 eingespiegelt und über die Abstrahlfläche 7 abgestrahlt, so dass es durch die Wandung 5 in das Medium 20 einstrahlt. Gleichzeitig wird der Bioreaktor 2 für die Reaktoraufnahme 9 in oszillierende Schwingungen versetzt. Über Befestigungsmittel 21 lässt sich der Bioreaktor 2 an der Reaktoraufnahme 9 fixieren.

## Patentansprüche

1. Bioreaktoranordnung (1), mit
einem Bioreaktor (2), der als ein Behälter mit einer transparenten Wandung (5) ausgebildet ist und
einem Beleuchtungskörper (3) zur Beleuchtung eines in einem Reaktorinnenraum (6) angeordneten Mediums (20),
wobei der Beleuchtungskörper (3)
mindestens eine Abstrahlfläche (7) aufweist, über die elektromagnetische Strahlung, wie Licht, abstrahlbar ist, und die elektromagnetische Strahlung über mindestens eine Stirnfläche (15, 16) des Beleuchtungskörpers in den Reaktorinnenraum (6) einspiegelbar ist,
**dadurch gekennzeichnet,**
**dass** die mindestens eine Stirnfläche (15, 16) als quer zum Beleuchtungskörper (3) verlaufender Durchbruch (13, 14) ausgebildet ist, in welchem eine Lichtleiste (17) mit einer Mehrzahl von LED-Lichtquellen (18) angeordnet ist und dass der Beleuchtungskörper (3) außerhalb des Reaktorinnenraumes (6) benachbart zu mindestens einem Teilbereich der transparenten Wandung (5) angeordnet ist und mit seiner Abstrahlfläche (7) eine Auflage- oder Stützfläche (8) für den Bioreaktor (2) bildet.

2. Bioreaktoranordnung nach Anspruch 1,
**dadurch gekennzeichnet,**
**dass** der Beleuchtungskörper (3) Aussparungen aufweist zur Durchführung von Sensoren, Anschlüssen oder Rührern zum Bioreaktor (2).

3. Bioreaktoranordnung nach Anspruch 1 oder 2,
**dadurch gekennzeichnet,**
**dass** der Beleuchtungskörper (3) an einer Reaktoraufnahme (9) einer Schüttelvorrichtung (4) angeordnet ist.

4. Bioreaktoranordnung nach einem der Ansprüche 1 bis 3,
**dadurch gekennzeichnet,**
**dass** der Bioreaktor (2) als ein flexibler Kunststoffbeutel zum Einmalgebrauch ausgebildet ist.

5. Bioreaktoranordnung nach einem der Ansprüche 1 bis 4,
**dadurch gekennzeichnet,**
**dass** der Beleuchtungskörper (3) als eine ebene Platte ausgebildet ist.

6. Bioreaktoranordnung nach einem der Ansprüche 1 bis 5,
**dadurch gekennzeichnet,**
**dass** der Beleuchtungskörper (3) in seinem Inneren lichtstreuende Partikel (19) aufweist.

7. Bioreaktoranordnung nach einem der Ansprüche 1 bis 6,
**dadurch gekennzeichnet,**
**dass** die dem Bioreaktor (2) zugewandte Abstrahlfläche (7) eine lichtstreuende Struktur aufweist.

8. Bioreaktoranordnung nach einem der Ansprüche 1 bis 7,
**dadurch gekennzeichnet,**
**dass** der Beleuchtungskörper (3) aus einem Acrylglas ausgebildet ist.

9. Schüttelvorrichtung (4) mit einer in Schwingungen versetzbaren Reaktoraufnahme (9) für einen als flexiblen Behälter mit einer transparenten Wandung (5) ausgebildeten Bioreaktor (2),
**dadurch gekennzeichnet,**
**dass** die Reaktoraufnahme (9) einen flächigen Beleuchtungskörper (3) aufweist, dessen Abstrahlfläche (7) für den aufzunehmenden Bioreaktor (2) eine Auflage- oder Stützfläche (8) bildet, über die elektromagnetische Strahlung, wie Licht, abstrahlbar ist, und die elektromagnetische Strahlung über mindestens eine Stirnfläche (15, 16) des Beleuchtungskörpers (3)in den Reaktorinnenraum (6) des Bioreaktors (2) einspiegelbar ist und dass die mindestens eine Stirnfläche (15, 16) als quer zum Beleuchtungskörper (3) verlaufender Durchbruch (13, 14) ausgebildet ist, in welchem eine Lichtleiste (17) mit einer Mehrzahl von LED-Lichtquellen (18) angeordnet ist.

10. Verfahren zum Bestrahlen eines Mediums in einem Bioreaktor (2) mit transparenter Wandung (5) mit elektromagnetischer Strahlung, wie Licht, der in einer Schüttelvorrichtung (4) in Schüttelbewegungen versetzt wird,
**dadurch gekennzeichnet,**
**dass** der Bioreaktor (2) in eine Reaktoraufnahme (9) der Schüttelvorrichtung (4) so eingesetzt wird, dass mindestens ein Teil seiner Wandung (5) an einer von einer Abstrahlfläche (7) eines flächigen Beleuchtungskörpers (3) gebildeten Auflage- oder Stützfläche (8) der Reaktoraufnahme (9) anliegt, und
**dass** in mindestens eine Stirnfläche (15, 16) des Beleuchtungskörpers (3) elektromagnetische Strahlung, wie Licht, eingespiegelt wird, die über die Abstrahlfläche (7) und die Wandung (5) des Bioreaktors (2) in das in dem Reaktorinnenraum (6) angeordnete Medium (20) einstrahlt wird und wobei die mindestens eine Stirnfläche (15, 16) als quer zum Beleuchtungskörper (3) verlaufender Durchbruch (13, 14) ausgebildet wurde, in welchem eine Lichtleiste (17) mit einer Mehrzahl von LED-Lichtquellen (18) angeordnet wurde.

11. Verfahren nach Anspruch 10,
**dadurch gekennzeichnet,**
**dass** die Wellenlänge und die Intensität der elektromagnetischen Strahlung, wie Licht, geregelt werden.

12. Verfahren nach Anspruch 11,
**dadurch gekennzeichnet,**
**dass** für die Regelung der Intensität der Einstrahlung die Biomassebildung erfasst wird.

## Claims

1. Bioreactor arrangement (1) comprising
a bioreactor (2), which is constructed as a container with a transparent wall (5) and
an illuminating body (3) for illuminating a medium (20) arranged in the reactor interior (6), wherein the illuminating body (3) comprises at least one irradiation surface (7) by way of which electromagnetic radiation, such as light, can be irradiated, and the electromagnetic radiation can be reflected by way of at least one end surface (15, 16) of the illuminating body into the reactor interior (6),
**characterised in that**
the at least one end surface (15, 16) is constructed as a passage (13, 14), which extends transversely to the illuminating body (3) and in which a light strip (17) with a plurality of LED light sources (18) is arranged,
and the illuminating body (3) is arranged outside the reactor interior (6) adjacent to at least one sub-region of the transparent wall (5) and together with its irradiation surface (7) forms a rest surface or support surface (8) for the bioreactor (2).

2. Bioreactor arrangement according to claim 1, **characterised in that** the illuminating body (3) has cut-outs for the passage of sensors, terminals or stirrers for the bioreactor (2).

3. Bioreactor arrangement according to claim 1 or 2, **characterised in that** the illuminating body (3) is arranged at a reactor mount (9) of an agitating device (4).

4. Bioreactor arrangement according to any one of claims 1 to 3, **characterised in that** the bioreactor (2) is constructed as a single-use flexible plastics material bag.

5. Bioreactor arrangement according to any one of claims 1 to 4, **characterised in that** the illuminating body (3) is constructed as a flat plate.

6. Bioreactor arrangement according to any one of claims 1 to 5, **characterised in that** the illuminating body (3) has light-dispersing particles (19) in its interior.

7. Bioreactor arrangement according to any one of claims 1 to 6, **characterised in that** the irradiation surface (7) facing the bioreactor (2) has a light-dispersing structure.

8. Bioreactor arrangement according to any one of claims 1 to 7, **characterised in that** the illuminating body (3) is constructed from an acrylic glass.

9. Agitating device (4) with a reactor mount (9), which can be set into oscillation, for a bioreactor (2) constructed as a flexible container with a transparent wall (5),
**characterised in that**
the reactor mount (9) has a flat illuminating body (3), the irradiation surface (7) of which forms for the bioreactor (2) to be mounted, a rest or support surface (8) by way of which electromagnetic irradiation, such as light, can be irradiated, and the electromagnetic irradiation can be reflected by way of at least one end surface (15, 16) of the illuminating body (3) into the reactor interior (6) of the bioreactor (2),
and the at least one end surface (15, 16) is constructed as a passage (13, 14), which extends transversely to the illuminating body (3) and in which a light-strip (17) with a plurality of LED light sources (18) is arranged.

10. Method of irradiating a medium in a bioreactor (2) with a transparent wall (5) with electromagnetic radiation, such as light, which is set into agitating movement in an agitating device (4),
**characterised in that**
the bioreactor (2) is so inserted into a reactor mount (9) of the agitating device (4) that at least a part of its wall (5) bears against a rest surface or support surface (8), which is formed by an irradiation surface (7) of a flat illuminating body (3), of the reactor mount (9), and in at least one end surface (15, 16) of the illuminating body (3) electromagnetic radiation, such as light, is reflected in, which radiation is radiated by way of the irradiation surface (7) and the wall (5) of the bioreactor (2) into the medium (20) arranged in the reactor interior (6), and wherein the at least one end surface (15, 16) was constructed as a passage (13, 14), which extends transversely to the illuminating body (3) and in which a light strip (17) with a plurality of LED light sources (18) was arranged.

11. Method according to claim 10, **characterised in that** the wavelength and the intensity of the electromagnetic radiation, such as light, are regulated.

12. Method according to claim 11, **characterised in that** biomass formation is detected for regulation of the intensity of the inward radiation.

## Revendications

1. Système de bioréacteur (1), comprenant
un bioréacteur (2), qui est réalisé sous la forme d'un récipient présentant une paroi transparente (5) et un corps d'éclairage (3) destiné à éclairer un milieu (20) situé dans un espace intérieur de réacteur (6),
dans lequel le corps d'éclairage (3) comprend au moins une surface d'émission (7) par l'intermédiaire de laquelle un rayonnement électromagnétique, tel que la lumière, peut être émis, et le rayonnement électromagnétique pouvant être réfléchi dans l'espace intérieur de réacteur (6) sur toute l'étendue d'au moins une surface frontale (15, 16) du corps d'éclairage,
**caractérisé en ce que**
ladite au moins une surface frontale (15, 16) est réalisée sous la forme d'un passage (13, 14) qui s'étend transversalement au corps d'éclairage (3), dans lequel est agencée une réglette lumineuse (17) présentant une pluralité de sources de lumière à DEL (18)
et **en ce que** le corps d'éclairage (3) est agencé à l'extérieur de l'espace intérieur de réacteur (6) au voisinage d'au moins une zone partielle de la paroi transparente (5) et forme avec sa surface d'émission (7) une surface de support ou d'appui (8) pour le bioréacteur (2).

2. Système de bioréacteur selon la revendication 1,
**caractérisé**
**en ce que** le corps d'éclairage (3) comprend des évidements permettant le passage de capteurs, de raccords ou d'agitateurs vers le bioréacteur (2).

3. Système de bioréacteur selon la revendication 1 ou 2,
**caractérisé**
**en ce que** le corps d'éclairage (3) est agencé sur un logement de réacteur (9) d'un secoueur (4).

4. Système de bioréacteur selon l'une quelconque des revendications 1 à 3,
**caractérisé**
**en ce que** le bioréacteur (2) est réalisé sous la forme d'un sachet flexible en matière plastique à usage unique.

5. Système de bioréacteur selon l'une quelconque des revendications 1 à 4,
**caractérisé**
**en ce que** le corps d'éclairage (3) est réalisé sous la forme d'une plaque plate.

6. Système de bioréacteur selon l'une quelconque des revendications 1 à 5,
**caractérisé**
**en ce que** le corps d'éclairage (3) comprend dans sa partie intérieure des particules (19) dispersant la lumière.

7. Système de bioréacteur selon l'une quelconque des revendications 1 à 6,
**caractérisé**
**en ce que** la surface d'émission (7) tournée vers le bioréacteur (2) comprend une structure dispersant la lumière.

8. Système de bioréacteur selon l'une quelconque des revendications 1 à 7,
**caractérisé**
**en ce que** le corps d'éclairage (3) est fait d'un verre acrylique.

9. Secoueur (4) comprenant un logement de réacteur (9) pouvant être soumis à des vibrations pour un bioréacteur (2) réalisé sous la forme d'un récipient flexible présentant une paroi transparente (5),
**caractérisé**
**en ce que** le logement de réacteur (9) comprend un corps d'éclairage plat (3) dont la surface d'émission (7) forme une surface de support ou d'appui (8) pour le bioréacteur (2) à loger, par l'intermédiaire de laquelle un rayonnement électromagnétique, tel que la lumière, peut être émis, et le rayonnement électromagnétique peut être réfléchi dans l'espace intérieur de réacteur (6) du bioréacteur (2) sur toute l'étendue d'au moins une surface frontale (15, 16) du corps d'éclairage (3)
et **en ce que** ladite au moins une surface frontale (15, 16) est réalisée sous la forme d'un passage (13, 14) qui s'étend transversalement au corps d'éclairage (3), dans lequel est agencée une réglette d'éclairage (17) présentant une pluralité de sources de lumière à DEL (18).

10. Procédé d'exposition d'un milieu dans un bioréacteur (2) présentant une paroi transparente (5) à un rayonnement électromagnétique, tel que la lumière, qui est soumis à des secousses dans un secoueur (4),
**caractérisé**
**en ce que** le bioréacteur (2) est inséré dans un logement de réacteur (9) du secoueur (4), de sorte qu'au moins une partie de sa paroi (5) s'applique contre une surface de support ou d'appui (8) du logement de réacteur (9) formée par une surface d'émission (7) d'un corps d'éclairage (3) plat, et
**en ce qu'**un rayonnement électromagnétique, tel que la lumière, est réfléchi dans au moins une surface frontale (15, 16) du corps d'éclairage (3), est projeté sur toute l'étendue de la surface d'émission (7) et de la paroi (5) du bioréacteur (2) dans le milieu (20) situé dans l'espace intérieur de réacteur (6) et dans lequel ladite au moins une surface frontale (15, 16) a été réalisée sous la forme d'un passage (13, 14) qui s'étend transversalement au corps d'éclairage (3), dans lequel une réglette d'éclairage (17) présentant une pluralité de sources de lumière à DEL (18) a été agencée.

11. Procédé selon la revendication 10,
**caractérisé**
**en ce que** la longueur d'onde et l'intensité du rayonnement électromagnétique, tel que la lumière, sont régulées.

12. Procédé selon la revendication 11,
**caractérisé**
**en ce que** pour la régulation de l'intensité de la projection, la formation de la biomasse est détectée.
